(19) Europäisches Patentamt / European Patent Office / Office européen des brevets

(11) **EP 4 559 492 A1**

(12) **EUROPEAN PATENT APPLICATION**

(43) Date of publication:
**28.05.2025   Bulletin 2025/22**

(21) Application number: **24214463.2**

(22) Date of filing: **21.11.2024**

(51) International Patent Classification (IPC):
**A61M 1/36** $^{(2006.01)}$        **A61M 1/38** $^{(2006.01)}$

(52) Cooperative Patent Classification (CPC):
**A61M 1/3693; A61M 1/02; A61M 1/38;**
A61M 1/36224; A61M 1/36225; A61M 2202/0427;
A61M 2205/3306; A61M 2205/3327;
A61M 2205/3334; A61M 2205/3365;
A61M 2230/207

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **22.11.2023   US 202363602335 P**

(71) Applicant: **Fenwal, Inc.
Lake Zurich, IL 60047 (US)**

(72) Inventors:
• **KUSTERS, Benjamin E.
Lake Zurich, 60047 (US)**
• **MIN, Kyungyoon
Lake Zurich, 60047 (US)**

(74) Representative: **Maikowski & Ninnemann
Patentanwälte Partnerschaft mbB
Postfach 15 09 20
10671 Berlin (DE)**

(54) **METHOD FOR PREDICTING WHOLE BLOOD PLATELET COUNT**

(57)    Blood processing systems, devices, and methods including a durable hardware component, a single use fluid flow circuit, and a controller configured to estimate the concentration of platelets in a donor's circulating blood.

FIG. 1

**EP 4 559 492 A1**

**EP 4 559 492 A1**

## Description

<u>Field of Disclosure</u>

The present disclosure relates generally to systems, devices, and methods for processing blood. More particularly, the disclosure relates generally to systems, devices, and methods for separating platelets from whole blood. Additionally, the disclosure relates to systems, devices, and methods for estimating the concentration of platelets in a donor's circulating blood during a blood processing procedure.

<u>Background</u>

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a human donor, because potentially less time is needed for the donor's body to return to pre-donation levels, and donations can be made at more frequent intervals than when whole blood is collected. This increases the overall supply of blood constituents, such as plasma and platelets, made available for transfer and/or therapeutic treatment.

Estimating a donor's circulating platelet concentration is an important part of any platelet collection procedure. The donor's platelet concentration is needed for the system to calculate the number of platelets collected (yield) and for system safeguards to ensure the donor's platelet count does not drop below acceptable limits such as 100e3 platelets/pL. Currently, blood processing systems apply complex mathematical algorithms to estimate collection yields and donor concentrations based on a donor platelet pre-count entered into the system prior to the start of collection. Although effective, some errors exist and a major limitation of requiring a donor pre-count is present. Accordingly, it would be desirable to carry out a safe platelet collection without the need for a donor platelet pre-count measurement.

Therefore, there exists a need for devices, systems, and methods to estimate the platelet concentration within a donor during a blood processing procedure.

<u>Summary</u>

There are several aspects of the present subject matter which may be embodied separately or together in the devices, systems, and methods described and/or claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto or later amended. For purposes of this description and claims, unless otherwise expressly indicated, "blood" is intended to include whole blood and blood components, such as concentrated red cells, plasma, platelets and white cells, whether with or without anticoagulant or additives.

In one aspect, a system for collecting platelets is provided. The system includes a reusable hardware unit, wherein the reusable hardware unit comprises a separator, a disposable fluid circuit configured to be associated with the reusable hardware unit, and a controller configured to estimate a platelet concentration of a donor's circulating blood.

In another aspect, a method for processing whole blood from a donor is provided. The method includes collecting whole blood from a donor, separating the collected whole blood into its constituent blood components including separated platelets, collecting the separated platelets, and estimating a concentration of platelets of a donor's blood during processing.

These and other aspects of the present subject are set forth in the following detailed description of the accompanying drawings.

<u>Brief Description of the Drawings</u>

FIG. 1 is a perspective view of an example of a reusable blood separation device.
FIG 2 is a schematic view of a disposable fluid circuit associated with a blood separation device.
FIG. 3 is a top plan view of an exemplary cassette of a fluid flow circuit in association with the blood separation device shown in FIG. 1.
FIG. 4 is a flow chart showing a method of determining the concentration of platelets in a donor's circulating blood.

2

Detailed Description of the Embodiments

The embodiments disclosed herein are for the purpose of providing an exemplary description of the present subject matter. They are, however, only exemplary and not exclusive, and the present subject matter may be embodied in various forms. Therefore, specific details disclosed herein are not to be interpreted as limiting the subject matter as defined in the accompanying claims.

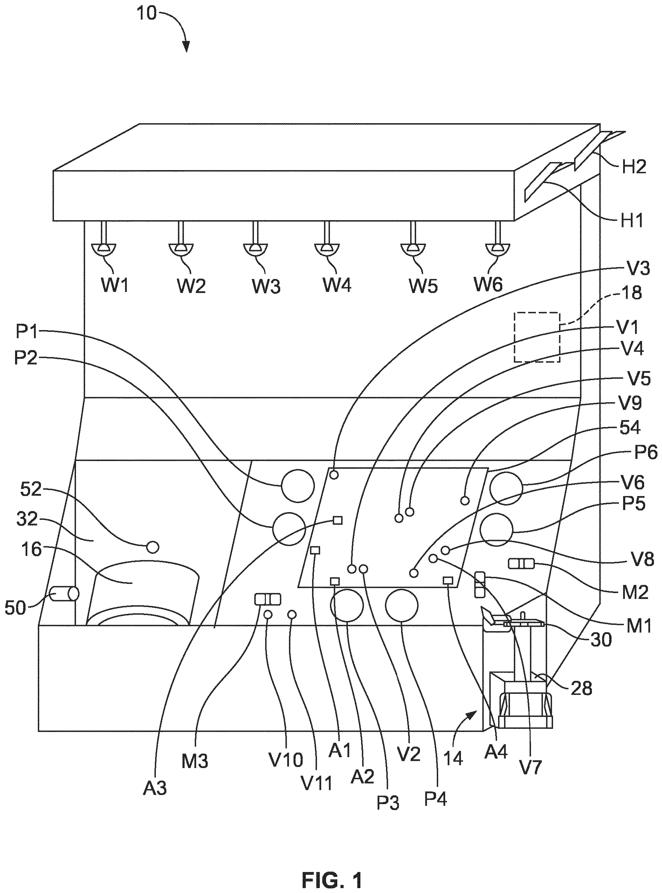

FIGS. 1-3 show components of a blood or fluid separation system that embodies various aspects of the present subject matter. While the system may be referred to herein as a "blood processing system" or a "blood separation system," it should be understood that systems according to the present disclosure can be used for processing a variety of fluids, which may include bodily fluids and non-bodily fluids. In particular, the blood processing system may be configured to separate whole blood into its constituent components including a concentrated platelet product. In an embodiment, the blood processing system is a system configured to separate and collect a platelet product from whole blood.

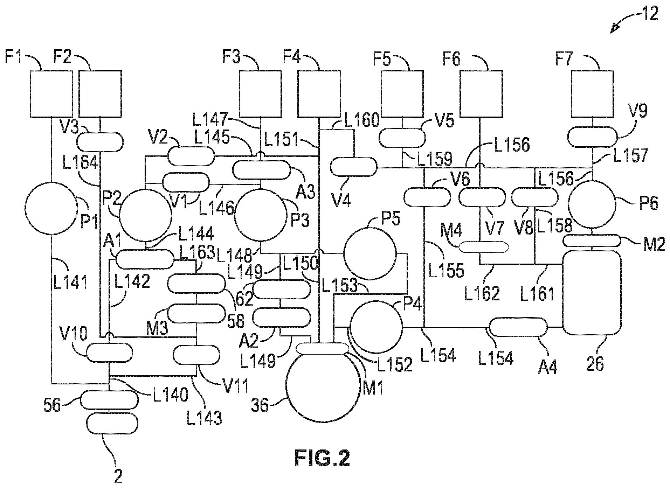

Generally, the system may include two principal components, a durable and reusable hardware unit (i.e., blood separation device) 10 (FIG. 1) and a disposable fluid flow circuit 12 (FIG. 2). The blood separation device 10 includes a spinning membrane separator drive unit 14, a centrifuge or centrifugal separator 16, additional components that control fluid flow through the disposable fluid flow circuit 12, and a controller 18, which governs the operation of the other components of the blood separation device 10 to perform a blood processing and collection procedure selected by the operator, as will be described in greater detail.

Turning to FIG. 1, the figure shows an example of a reusable blood separation device 10. The blood separation device 10 is configured as a durable item that is capable of long-term use. It will be understood that the blood separation device 10 is merely exemplary of one possible configuration and that blood separation devices according to the present disclosure may be differently configured. Blood separation device 10 can be the blood separation device disclosed in U.S. Patent No. 11,465,160, filed on February 22, 2019, which is hereby incorporated by reference herein in its entirety, or any other blood separation configured to collect a platelet product known in the art.

In an embodiment, blood separation device 10 may be a portable device configured to rest on an elevated, generally horizontal support surface (e.g., a countertop or a tabletop), but it is also within the scope of the present disclosure for the device 10 to include a support base to allow the device 10 to be appropriately positioned and oriented when placed onto a floor or ground surface. While it may be advantageous for the blood separation device 10 to be portable, it is also within the scope of the present disclosure for the blood separation device 10 to be installed in a single location and remain in that location for an extended period of time. If the blood separation device is provided as a fixture, it may be provided with more components and functionality than a more portable version.

As shown, the blood separation device 10 includes a spinner support or spinning membrane separator drive unit 14 for accommodating a generally cylindrical spinning membrane separator 26 (FIG. 2) of the fluid flow circuit 12. U.S. Patent No. 5,194,145, incorporated herein in its entirety, describes an exemplary spinning membrane separator drive unit that would be suitable for incorporation into the blood separation device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the blood separation device 10.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may be as described in previously incorporated U.S. Patent No. 11,465,160 and may vary without departing from the scope of the present disclosure.

Regardless of the mechanism by which the spinning membrane separator drive unit 14 spins the component or components of the spinning membrane separator 26, the component or components of the spinning membrane separator 26 is preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

Separation device 10 may also include a centrifugal separator 16. In an embodiment, separation device 10 includes a centrifuge compartment 32, which may house the centrifugal separator 16 and/or the other components of the centrifugal separator 16. The centrifuge compartment 32 may include a lid (not shown) that is opened to insert and remove a centrifugal separation chamber 36 of the fluid flow circuit 12. During a separation procedure, the lid may be closed with the centrifugal separation chamber 36 positioned within the centrifuge compartment 32, as the centrifugal separation chamber 36 is spun or rotated about an axis under the power of an electric drive motor or rotor of the centrifugal separator 16.

Components of an interface monitoring system may be positioned within the centrifuge compartment 32 to oversee separation of blood within the centrifugal separation chamber 36. The interface monitoring system may include a light source 50 and a light detector 52, which is positioned and oriented to receive at least a portion of the light emitted by the light source 50. Preferably, the light source 50 and the light detector 52 are positioned on stationary surfaces of the centrifuge compartment 32, but it is also within the scope of the present disclosure for one or both to be mounted to a movable component of the centrifugal separator 16 (e.g., a yoke member, which rotates at a one omega speed). A more detailed description of an example of an interface monitoring system is disclosed in previously incorporated U.S. Patent No. 11,465,160.

In addition to the spinning membrane separator drive unit 14 and the centrifugal separator 16, the blood separation device 10 may include other components compactly arranged to aid blood processing.

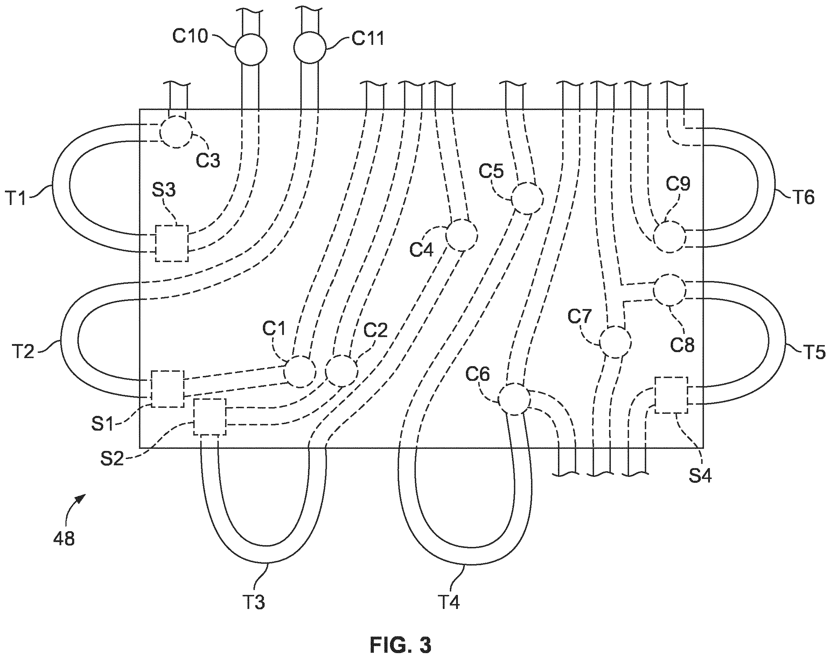

For example, blood separation device 10 includes a cassette station 54, which accommodates a cassette 48 of the fluid flow circuit 12 (FIG. 3). In one embodiment, the cassette station 54 is similarly configured to the cassette station of U.S. Patent No. 5,868,696 (which is incorporated herein by reference), but is adapted to include additional components and functionality. The illustrated cassette station 54 includes a plurality of clamps or valves V1-V9 (FIG. 1), which move between a plurality of positions (e.g., between a retracted or lowered position and an actuated or raised position) to selectively contact or otherwise interact with corresponding valve stations C1-C9 of the cassette 48 of the fluid flow circuit 12. Depending on the configuration of the fluid flow circuit 12, its cassette 48 may not include a valve station C1-C9 for each valve V1-V9 of the cassette station 54, in which case fewer than all of the valves V1-V9 will be used in a separation procedure.

In the actuated position, a valve V1-V9 engages the associated valve station C1-C9 to prevent fluid flow through that valve station C1-C9 (e.g., by closing one or more ports associated with the valve station C1-C9, thereby preventing fluid flow through that port or ports). In the retracted position, a valve V1-V9 is disengaged from the associated valve station C1-C9 (or less forcefully contacts the associated valve station C1-C9 than when in the actuated position) to allow fluid flow through that valve station C1-C9 (e.g., by opening one or more ports associated with the valve station C1-C9, thereby allowing fluid flow through that port or ports). Additional clamps or valves V10 and V11 may be positioned outside of the cassette station 54 to interact with portions or valve stations C10 and C11 (which may be lengths of tubing) of the fluid flow circuit 12 to selectively allow and prevent fluid flow therethrough. The valves V1-V9 and corresponding valve stations C1-C9 of the cassette station 54 and cassette 48 may be differently configured and operate differently from the valves V10 and V11 and valve stations C10 and C11 that are spaced away from the cassette station 54.

The cassette station 54 may be provided with additional components, such as pressure sensors A1-A4, which interact with sensor stations S1-S4 of the cassette 48 to monitor the pressure at various locations of the fluid flow circuit 12. For example, if the blood source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensor A1-A4 that are indicative of the pressure within the fluid flow circuit 12 and, if a signal indicates a low- or high-pressure condition, the controller 18 may initiate an alarm or error condition to alert an operator to the condition and/or to attempt to bring the pressure to an acceptable level without operator intervention.

The blood separation device 10 may also include a plurality of pumps P1-P6 to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696, which is incorporated by reference herein in its entirety. Each pump P1-P6 engages a different tubing loop T1-T6 extending from a side surface of the cassette 48 (FIG. 3) and may be selectively operated under command of the controller 18 to cause fluid to flow through a portion of the fluid flow circuit 12, as will be described in greater detail. In one embodiment, all or a portion of the cassette station 54 may be capable of translational motion in and out of the separation device 10 to allow for automatic loading of the tubing loops T1-T6 into the associated pump P1-P6.

The illustrated blood separation device 10 also includes a centrifuge fluid sensor M1 for determining one or more properties of a fluid flowing into or out of a centrifugal separation chamber 36 mounted within the centrifugal separator 16. If the fluid flowing into the centrifuge separation chamber 36 is whole blood (which may include anticoagulated whole blood), the sensor M1 may be configured to determine the hematocrit of the blood flowing into the centrifuge separation chamber 36. If the fluid flowing out of the centrifuge separation chamber 36 is platelet-rich plasma, the sensor M1 may be configured to determine the platelet concentration of platelet-rich plasma flowing into the spinning membrane separator 26. The sensor M1 may detect the one or more properties of a fluid by optically monitoring the fluid as it flows through tubing of the fluid flow circuit 12 or by any other suitable approach. The controller 18 may receive signals from the sensor M1 that are indicative of the one or more properties of fluid flowing in fluid flow circuit 12 and use the signals to optimize the separation procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is incorporated herein by reference), but it should be understood that a different approach may also be employed for monitoring hematocrit and/or platelet concentration of fluid flowing into the spinning membrane separator 26.

The illustrated blood separation device 10 further includes a spinner outlet sensor M2, which accommodates tubing of the fluid flow circuit 12 that flows a separated blood component out of the spinning membrane separator 26. The spinner outlet sensor M2 monitors the fluid to determine one or more properties of the fluid, and may do so by optically monitoring the fluid as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is incorporated herein by reference) or by any other suitable device and/or method.

The illustrated blood separation device 10 also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

In an embodiment, separation device 10 may optionally include platelet concentrate sensor M4. Sensor M4 may be incorporated into the separation device 10 or may be an independent sensor. Platelet concentrate sensor M4 may be configured to determine the concentration of a concentrated platelet product exiting the spinning membrane 26. The sensor M4 may detect the concentration of the concentrated platelet product by optically monitoring the fluid as it flows through tubing of the fluid flow circuit 12 or by any other suitable approach.

Separation device 10 may also include a plurality of weight scales W1-W6 (six are shown, but more or fewer may be provided), each of which may support one or more fluid containers of the fluid flow circuit 12. The fluid containers receive blood components separated during processing or intravenous fluids or additive fluids. Each weight scale W1-W6 transmits to the controller 18 a signal that is indicative of the weight of the fluid within the associated fluid container to track the change of weight during the course of a procedure. This allows the controller 18 to process the incremental weight changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated separation device 10 is also provided with a plurality of hooks or supports H1 and H2 that may support various components of the fluid flow circuit 12 or other suitably sized and configured objects.

The blood separation device 10 includes a controller 18, which is suitably configured and/or programmed to control operation of the blood separation device 10. In one embodiment, the controller 18 may be the controller disclosed in previously incorporated U.S. Patent No. 11,465,160. In one embodiment, the controller 18 may be mounted inside the separation device 10. Additionally, controller 18 may be mounted inside the separation device 10 adjacent to or incorporated into an operator interface station (e.g., a touchscreen) (not shown). In other embodiments, the controller 18 and operator interface station may be associated with or incorporated into a separate device that is connected (either physically, by a cable or the like, or wirelessly) to the blood separation device 10.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

Controller 18 is configured and/or programmed to execute at least one blood processing application. In particular, controller 18 is configured and/or programmed to carry out a platelet collection procedure. Controller 18 may also be configured and/or programmed to execute additional or alternative blood processing procedures, for example, a double unit red blood cell collection procedure, a plasma collection procedure, a plasma/red blood cell collection procedure, a red blood cell/platelet/plasma collection procedure, a platelet collection procedure, and a platelet/plasma collection procedure without departing from the scope of the present disclosure.

More particularly, in carrying out any one of these blood processing applications, the controller 18 is configured and/or programmed to control one or more of the following tasks: drawing blood into a fluid flow circuit 12 mounted to the blood separation device 10, conveying blood through the fluid flow circuit 12 to a location for separation (i.e., into a spinning membrane separator 26 or centrifugal separation chamber 36 of the fluid flow circuit 12), separating the blood into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a donor from which the blood was originally drawn).

This may include instructing the spinning membrane separator drive unit 14 and/or the centrifugal separator 16 to operate at a particular rotational speed and instructing a pump P1-P6 to convey fluid through a portion of the fluid flow circuit 12 at a particular flow rate. Hence, while it may be described herein that a particular component of the blood separation device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the blood separation device 10 (e.g., the pressure sensors A1-A4 and sensors M1-M4) to monitor various aspects of the operation of the blood separation device 10 and characteristics of the blood and separated blood components as they flow through the fluid flow circuit 12. For example, controller 18 may monitor a separation interface using an interface control module as described in previously incorporated U.S. Patent No. 11,465,160. If the operation of any of the components and/or one or more characteristics of the blood or separated blood components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

In accordance with the present disclosure, the controller 18 is configured to monitor and estimate the platelet concentration of a donor's circulating blood ($C_{donor}$) throughout a blood processing procedure, such as a platelet separation and collection procedure. The donor's platelet concentration is needed for the system to calculate the number of platelets collected (platelet yield) and for system safeguards to ensure the donor's platelet count does not drop below acceptable limits (e.g., 100e3 platelets/pL). By estimating the platelet concentration of a donor's circulating blood during a blood processing procedure, a platelet pre-count measurement is not required.

The controller 18 may determine $C_{donor}$ based on values obtained by the controller 18 from the various sensors and scales that are coupled to the controller, and other inputs associated with the system and the following equations:

$$C_{WBcent} = C_{PRP}*(1 - H_{Wbcent}) \qquad \text{[Equation 1]}$$

$$H_{Wbcent} = (H_{RBC}*Q_{RBC})/Q_{Wbcent} \qquad \text{[Equation 2]}$$

$$WB\% = Part\ WB/(Part\ WB + Part\ AC) \qquad \text{[Equation 3]}$$

$$C_{donor} = C_{Wbcent}*WB\% \qquad \text{[Equation 4]}.$$

As shown in Equation 4, above, $C_{donor}$ may be based on the concentration of platelets ($C_{Wbcent}$) in collected whole blood entering the centrifugal separator 16 and the percentage of whole blood (WB%) in a centrifuge inlet line L149 (shown in FIG. 2). In particular, $C_{donor}$ is determined by determining the product of $C_{Wbcent}$ and WB%.

$C_{Wbcent}$ is based on the platelet concentration in separated platelet rich plasma ($C_{PRP}$) exiting the centrifuge separation chamber 36 and the hematocrit of collected whole blood ($H_{Wbcent}$) entering the centrifuge separation chamber 36. In particular, $C_{Wbcent}$ is determined by Equation 1, such that $C_{PRP}$ is multiplied with the difference between 1 and $H_{Wbcent}$. In an embodiment, $C_{PRP}$ can be determined using optical based measurements of the platelet rich plasma exiting the centrifuge separation chamber 36. For instance, $C_{PRP}$ can be determined by sensor M1 of the separation device 10. In an embodiment, $C_{PRP}$ can be determined by optical measurements recorded by sensor M1 and a method for platelet concentration estimation, such as that described in U.S. Application Serial No. 18/101,275 filed on January 25, 2023, the disclosure of which is herein incorporated by reference in its entirety.

$H_{Wbcent}$ can be determined using the hematocrit of red blood cells ($H_{RBC}$) exiting the centrifuge separation chamber 36, the flow rate of red blood cells ($Q_{RBC}$) exiting the centrifuge separation chamber 36, and the flow rate of whole blood ($Q_{Wbcent}$) entering the centrifuge separation chamber 36. In particular, $H_{Wbcent}$ is determined using Equation 2, such that $H_{Wbcent}$ is the product of $H_{RBC}$ and $Q_{RBC}$ divided by $Q_{Wbcent}$. In an embodiment, $H_{RBC}$ can be assumed to be a constant empirically derived value for all procedures, for example, 85%. Alternatively, $H_{RBC}$ may be estimated by an optical sensor, such as sensor M1, or it may be determined by any other suitable method, such as using the centrifuge flow rates and centrifugation g-force, without departing from the scope of the disclosure. $Q_{RBC}$ can be determined based on the $Q_{WBcent}$ and the flow rate of platelet rich plasma ($Q_{PRP}$) exiting the centrifuge, such that $Q_{RBC}$ is the difference of $Q_{WBcent}$ and $Q_{PRP}$. $Q_{WBcent}$ and $Q_{PRP}$ may be known rates as determined by the pump rate of their respective pumps 3 and 5, pump rotation feedback, weight scale changes, or a combination of these flow rate monitoring methods. For example, $Q_{WBcent}$ and $Q_{PRP}$ may be pre-determined flow rates or may be determined by the controller 18 based on data from the weight scales and/or the optical system.

WB% can be determined by dividing the amount (e.g., volume or flow rate) of whole blood in an inlet line entering the centrifuge with the amount (volume or flow rate) of total fluid in the inlet line entering the centrifuge. The total fluid in the inlet line may include whole blood with an additive, such as an anticoagulant. In an embodiment, WB% can be determined using Equation 3, such that the number of parts of whole blood is divided by the total number of parts of whole blood and number of parts of anticoagulant in the inlet line entering the centrifuge. For example, the inlet line may include an anticoagulant ratio of 10:1, which is 10 parts of whole blood to 1 part of anticoagulant. Accordingly, because the collected whole blood is diluted with anticoagulant solution, $C_{donor}$ is higher than $C_{WBcent}$ in the collected whole blood entering the centrifuge.

To determine $C_{donor}$, Equations 1, 2, and 3 can be substituted into Equation 4 to produce Equation 5, below:

$$C_{donor} = C_{PRP}*WB\%*(1-((H_{RBC}*Q_{RBC})/Q_{WBcent}))  \quad \text{[Equation 5]}.$$

Alternatively, $C_{donor}$ can be based on the concentration of platelets ($C_{PC}$) in a platelet concentrate line L162 (shown in FIG. 2), rather than the platelet-rich plasma concentration ($C_{PRP}$). In this case, $C_{PRP}$ can be substituted with $C_{PC}$, the flow rate of platelet concentration ($Q_{PC}$) exiting a spinning membrane 26, and the flow rate of platelet-rich plasma ($Q_{PRP}$) entering the spinning membrane 26. The relationship between $C_{PRP}$ and the values of $C_{PC}$, $Q_{PC}$, and $Q_{PRP}$ are shown in Equation 6, below:

$$C_{PRP} = (C_{PC}*Q_{PC})/Q_{PRP}  \quad \text{[Equation 6]}.$$

$C_{PC}$ can be obtained by an optical measurement using a sensor M4 of separation device 10 or it may be determined by any other suitable method. $Q_{PC}$ and $Q_{PRP}$ may be pre-determined flow rates or may be determined by the controller 18 based on data from the weight scales and/or the optical system.

Accordingly, Equation 6 can be substituted into Equation 4 to determined $C_{donor}$, such that:

$$C_{donor} = WB\%*((C_{PC}*Q_{PC})/Q_{PRP})*(1-((H_{RBC}*Q_{RBC})/Q_{WBcent}))  \quad \text{[Equation 7]}.$$

Using Equations 5 or 7, the controller 18 may estimate $C_{donor}$ circulating in a donor's blood throughout a blood processing procedure. In an embodiment, controller can be pre-programmed to determine $C_{donor}$ using either Equation 5 or Equation 7. Alternatively, controller 18 may be programmed to determine $C_{donor}$ with either Equation 5 or Equation 7, and a user may input which method to use using a controller interface such as a touchscreen or keyboard. By determining the $C_{donor}$ of a donor's circulating blood throughout a blood processing procedure, a platelet pre-count does not need to be determined.

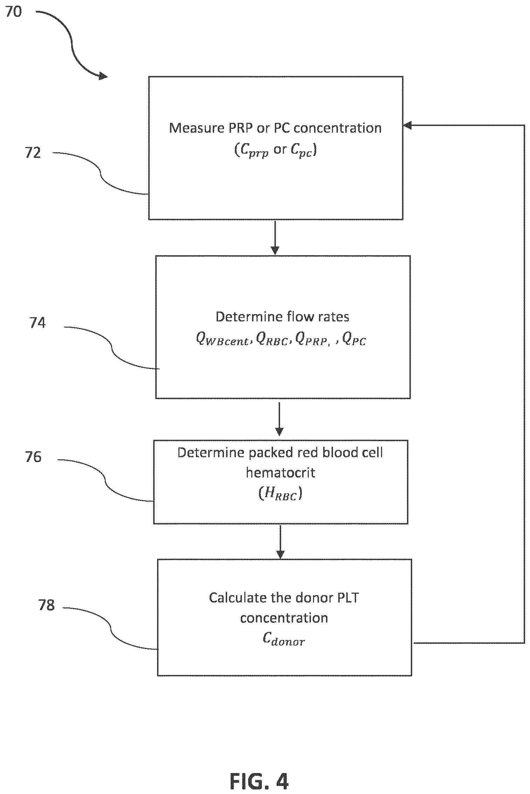

Controller 18 may be configured to continuously monitor $C_{donor}$ with method 70 as shown in FIG. 4. For example, the method may include steps 72-78 including, respectively: measuring the platelet rich plasma or platelet concentrate concentration ($C_{PRP}$ or $C_{PC}$, respectively), determining the flow rates $Q_{WBcent}$, $Q_{RBC}$, $Q_{PRP}$, and $Q_{PC}$, determining the hematocrit of packed red blood cells ($H_{RBC}$) exiting the centrifugal separation chamber 36, and calculating $C_{donor}$ using the equations described herein. In an embodiment, steps 72-78 may be executed sequentially, and restarted after step 78 (calculating $C_{donor}$). In another embodiment, steps 72-76 may be executed sequentially, in any order, or simultaneously. After executing steps 72-76, the controller may execute step 78, calculating the $C_{donor}$, and then restarting the method 70.

Additionally, by continuously monitoring $C_{donor}$, controller 18 can be programmed to initiate an alarm or to alert a user when $C_{donor}$ drops below a pre-determined limit, for instance 100e3/μL. The pre-determined limit may be pre-programmed into controller 18 or a user may input a limit into a controller interface (if provided) before initiating a procedure. In an embodiment, if $C_{donor}$ drops below a pre-determined limit, controller 18 may alert the user to end the procedure and/or automatically end the procedure. Additionally, the controller may be programmed to alert the user that a pre-determined limit is approaching. For example, if the pre-determined limit is 100e3/μL, the controller may be programmed to alert a user when $C_{donor}$ drops to 120e3/μL. In this instance, a user can adjust the target final platelet yield for the process.

As for the fluid flow circuit 12, it is intended to be a sterile, single use, disposable item. Before beginning a given blood processing and collection procedure, the operator loads various components of the fluid flow circuit 12 on the blood separation device 10. The controller 18 implements the procedure based upon preset protocols, taking into account other input from the operator. Upon completing the procedure, the operator removes the fluid flow circuit 12 from association with the blood separation device 10. The portions of the fluid flow circuit 12 holding the collected blood component or components (e.g., collection containers or bags) are removed from the separation device 10 and retained for storage, transfusion, or further processing. The remainder of the fluid flow circuit 12 may be removed from the separation device 10 and discarded.

A variety of different disposable fluid flow circuits may be used in combination with the blood separation device 10, with the appropriate fluid flow circuit depending on the separation procedure to be carried out using the system. Generally, the fluid flow circuit 12 includes a cassette 48 (FIG. 3), to which the other components of the fluid flow circuit 12 are connected by flexible tubing. The other components may include a plurality of fluid containers F1-F7 (for holding blood, a separated blood component, an intravenous fluid, or an additive solution), one or more blood source access devices (e.g., a phlebotomy needle or a connector for accessing blood within a fluid container), and a spinning membrane separator 26 and/or a centrifugal separation chamber 36.

The cassette 48 provides a centralized, programmable, integrated platform for all the pumping and many of the valving functions required for a given blood processing procedure. In one embodiment, the cassette 48 is similarly configured to the cassette of U.S. Patent No. 5,868,696, but is adapted to include additional components (e.g., more tubing loops T1-T6) and functionality.

In use, the cassette 48 is mounted to the cassette station 54 of the blood separation device 10, with a flexible diaphragm of the cassette 48 placed into contact with the cassette station 54. The flexible diaphragm overlays an array of interior cavities formed by the body of the cassette 48. The different interior cavities define sensor stations S1-S4, valve stations C1-C9, and a plurality of flow paths. The side of the cassette 48 opposite the flexible diaphragm may be sealed by another flexible diaphragm or a rigid cover, thereby sealing fluid flow through the cassette 48 from the outside environment.

Each sensor station S1-S4 is aligned with an associated pressure sensor A1-A4 of the cassette station 54, with each pressure sensor A1-A4 capable of monitoring the pressure within the associated sensor station S1-S4. Each valve station C1-C9 is aligned with an associated valve V1-V9, and may define one or more ports that allow fluid communication between the valve station C1-C9 and another interior cavity of the cassette 48 (e.g., a flow path). As described above, each valve V1-V9 is movable under command of the controller 18 to move between a plurality of positions (e.g., between a retracted or lowered position and an actuated or raised position) to selectively contact the valve stations C1-C9 of the cassette 48. In the actuated position, a valve V1-V9 engages the associated valve station C1-C9 to close one or more of its ports to prevent fluid flow therethrough. In the retracted position, a valve V1-V9 is disengaged from the associated valve station C1-C9 (or less forcefully contacts the associated valve station C1-C9 than when in the actuated position) to open one or more ports associated with the valve station C1-C9, thereby allowing fluid flow therethrough.

As described, a plurality of tubing loops T1-T6 extend from the side surface of the cassette 48 to interact with pumps P1-P6 of the blood separation device 10. In the illustrated embodiment, six tubing loops T1-T6 extend from the cassette 48 to be received by a different one of six pumps P1-P6, but in other embodiments, a procedure may not require use of all of the pumps P1-P6, in which case the cassette 48 may include fewer than six tubing loops. The different pumps P1-P6 may interact with the tubing loops T1-T6 of the cassette 48 to perform different tasks during a separation procedure, but in one embodiment, a different one of the pumps P1-P6 may be configured to serve as a source pump, an anticoagulant pump, a spinner pump, a separated component pump, an additive pump, and a replacement fluid pump. As will be described, certain procedures require fewer than all of the sensor stations, valve stations, and/or tubing loops illustrated in the exemplary cassette 48 of FIG. 3, such that it should be understood that the cassettes of different fluid flow circuits 12 may be differently configured (e.g., with fewer sensor stations, valve stations, and/or tubing loops) without departing from the scope of the present disclosure.

Additional tubing extends from the side surface of the cassette 48 to connect to the other components of the fluid flow circuit 12, such as the various fluid containers F1-F7, the spinning membrane separator 26 (if provided), and the centrifugal separation chamber 36 (if provided). The number and content of the various fluid containers F1-F7 depends upon the procedure for which the fluid flow circuit 12 is used.

Various additional components may be incorporated into the tubing leading out of the cassette 48 or into one of the cavities of the cassette 48. For example, as shown in FIG. 2, a manual clamp 56 may be associated with a line or lines leading to the blood source and/or fluid recipient, a return line filter 58 (e.g., a microaggregate filter) may be associated with a line leading to a fluid recipient, and/or an air trap 62 may be positioned on a line upstream of the centrifugal separation chamber 36. In an embodiment, based on the configuration of the fluid flow circuit 12 and cassette 48, certain valves may be substituted with clamps, and vice versa.

Exemplary Separation Procedure

The separation device 10 may be associated with a variety of fluid circuits configured for certain blood processing procedures. An exemplary blood separation procedure that may be carried out using systems and techniques according to the present disclosure will now be described.

Depending on the blood separation objectives, there is a suitable procedure for separating and collecting any combination of red blood cells, platelets, white blood cells, and (substantially cell-free) plasma. Accordingly, prior to processing, an operator selects the desired protocol (e.g., using an operator interface station, if provided), which informs the controller 18 of the manner in which it is to control the other components of the blood separation device 10 during the procedure.

If the blood source is a donor, the operator may proceed to enter various parameters, such as the donor gender/height/weight. In one embodiment, the operator also enters the target yield for the various blood components (which may also include entering a characteristic of the blood, such as a platelet pre-count) or some other collection control system (e.g., the amount of whole blood to be processed).

If there are any fluid containers (e.g., a platelet storage solution container) that are not integrally formed with the fluid flow circuit 12, they may be connected to the fluid flow circuit 12 (e.g., by piercing a septum of a tube of the fluid flow circuit 12 or via a luer connector), with the fluid flow circuit 12 then being mounted to the blood separation device 10 (including the fluid containers F1-F7 being hung from the weight scales W1-W6, as appropriate). An integrity check of the fluid flow circuit 12 may be executed by the controller 18 to ensure the various components are properly connected and functioning. Following a successful integrity check, the blood source is connected to the fluid flow circuit 12 (e.g., by phlebotomizing a donor), and the fluid flow circuit 12 may be primed (e.g., by saline pumped from a saline bag F2 by operation of one or more of the pumps P1-P6 of the blood separation device 10).

When the fluid flow circuit 12 has been primed, blood separation may begin. The stages of blood separation vary depending on the particular procedure.

According to one aspect of the present disclosure, the blood separation device 10 may be used to separate and collect platelets or both platelets and substantially cell-free plasma from blood. A blood separation device 10 according to the present disclosure may be used in combination with a fluid flow circuit 12.

FIG. 2 is a schematic view of an exemplary fluid flow circuit 12 having a single blood access device (e.g., a single needle that draws blood from and returns a separated blood component to the same location) that may be used to separate and collect only platelets or both platelets and plasma. The fluid flow circuit 12 includes a cassette 48 of the type described above and illustrated in FIG. 3, which connects the various components of the fluid flow circuit 12. The various connections amongst the components of the fluid flow circuit 12 are shown in FIG. 2, which also shows the fluid flow circuit 12 mounted to the blood separation device 10.

All of the various valves V1-V11 and pressure sensors A1-A4 of the blood separation device 10 are used in combination with the fluid flow circuit 12 of FIG. 2 for separating and collecting platelets or platelets and plasma, except for pressure sensor A3. As will be described, fluid may flow through the sensor station S3 associated with pressure sensor A3, but the pressure sensor A3 does not communicate with the controller 18 to monitor the pressure at any location within the fluid flow circuit 12. Additionally, as will be described, both the centrifugal separator 16 and the spinning membrane separator drive unit 14 are used in separating blood into platelets or platelets and plasma for collection.

In a first phase, blood is drawn into the fluid flow circuit 12 from a blood source 2. If the blood source 2 is a donor, then blood may be drawn into the fluid flow circuit 12 through a single needle that is connected to the cassette 48 by line L140. The line L140 may include a manual clamp 56 that may initially be in a closed position to prevent fluid flow through the line L140. When processing is to begin, an operator may move the manual clamp 56 from its closed position to an open position to allow fluid flow through the line L140.

The blood is drawn into the line L140 by the donor pump P2 of the blood separation device 10. Anticoagulant from the anticoagulant bag F1 may be drawn through line L141 under action of the anticoagulant pump P1 and added to the blood at a junction of lines L140 and L141.

In the illustrated embodiment, the valve V10 associated with valve station C10 of the fluid flow circuit 12 is open to allow blood to flow through lines L140 and L142 and a sensor station S1 associated with pressure sensor A1, while the valve V11 associated with valve station C11 is closed to prevent fluid flow through line L143. If the blood source is a living body (e.g., a donor), the pressure sensor A1 may communicate with the controller 18 to monitor the pressure within the vein of the blood source.

The cassette 48 includes two valve stations C1 and C2 downstream of the donor pump P2 and line L144, with the valve V2 associated with valve station C2 being closed to prevent flow through line L145 and the valve V1 associated with valve station C1 being open to allow flow through line L146. The blood flows through the line L146 associated with the open valve V1 to a junction, where a portion of the blood is directed through line L147 and the sensor station S3 associated with inactive pressure sensor A3 to the in-process bag F3 and the remainder is directed through line L148 toward the centrifuge pump P3, which controls the amount of blood that is directed to the centrifugal separation chamber 36 instead of the in-process bag F3. In particular, the flow rate of the donor pump P2 is greater than the flow rate of the centrifuge pump P3, with the difference therebetween being equal to the flow rate of blood into the in-process bag F3. The flow rates may be selected such that the in-process bag F3 is partially or entirely filled with blood at the end of the draw phase.

The blood pumped through line L148 by the centrifuge pump P3 passes through line L149, an air trap 62, and the sensor station S2 associated with pressure sensor A2 (which works in combination with the controller 18 of the blood separation device 10 to monitor the pressure in the centrifugal separation chamber 36) before reaching the centrifugal separation chamber 36 of the fluid flow circuit 12. The centrifugal separator 16 of the blood separation device 10 manipulates the centrifugal separation chamber 36 to separate the blood in the centrifugal separation chamber 36 into platelet-rich plasma and packed red blood cells, as described above. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L150 and flow through line L151 into the return bag F4. In an embodiment, the packed red blood cells travel through the centrifuge fluid sensor M1 as they exit the centrifugal separation chamber 36. The centrifuge fluid sensor M1 may detect the hematocrit of the packed red blood cells exiting the centrifugal separation chamber. White blood cells may be retained within the centrifugal separation chamber 36 or may exit with the red blood cells.

Platelet-rich plasma is drawn out of the centrifugal separation chamber 36 via line L152 by the combined operation of the recirculation and PRP pumps P5 and P4 of the blood separation device 10. The platelet-rich plasma travels through the centrifuge fluid sensor M1 and line L152 until it reaches a junction, which splits into lines L153 and L154. The centrifuge fluid sensor M1 may detect the concentration of platelets in the platelet-rich plasma. The recirculation pump P5 is associated with line L153 and redirects a portion of the platelet-rich plasma to a junction, where it mixes with blood in line L148 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5.

As the platelet-rich plasma drawn out of the centrifugal separation chamber 36 into line L153 by the recirculation pump P5 is immediately added back into the centrifugal separation chamber 36, the bulk or net platelet-rich plasma flow rate out of the centrifugal separation chamber 36 is equal to the flow rate of the PRP pump P4. Before reaching the spinning membrane separator 26, the portion of the platelet-rich plasma conveyed through line L154 by the PRP pump P4 passes through the sensor station S4 associated with pressure sensor A4. The pressure sensor A4 may monitor the pressure of the spinning membrane separator 26.

Line L154 has a junction, where it joins with line L155. The valve V6 associated with valve station C6 is closed to prevent fluid flow through the line L155, thereby directing the separated platelet-rich plasma to the spinning membrane separator 26. The valve V6 may be selectively opened to divert all or a portion of the platelet-rich plasma through line L155 and to the return bag F4, if necessary. An example would be at the start of a procedure when separation is initializing and platelets are not yet exiting the centrifugal separation chamber 36, in which case the fluid conveyed through line L154 by the PRP pump P4 could be diverted to the return bag F4.

The spinning membrane separator drive unit 14 of the blood separation device 10 operates the spinning membrane separator 26 to separate the platelet-rich plasma into two sub-components (i.e., platelet-poor plasma and platelet concentrate). In an embodiment, the spinning membrane separator drive unit 14 may rotate at approximately 1,500 rpm (or at a different speed, depending on the flow rates of fluid into and out of the spinning membrane separator 26) to separate platelet-rich plasma entering, for example, the bottom portion of the spinning membrane separator 26 into plasma and platelets, according to the principles described above.

Plasma is pumped out of the spinning membrane separator 26 via line L156 by the plasma pump P6 of the blood separation device 10. Valves V9, V8, V6, and V5 associated with valve stations C9, C8, C6, and C5 (respectively) are closed to prevent flow through lines L157, L158, L155, and L159, thereby directing the separated plasma along lines L156 and L160, through the valve station C4 associated with open valve V4, and into the return bag F4 (with the separated red blood cells). On the way to the return bag F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L161. In an embodiment, there is no pump associated with line L161, so instead the flow rate at which the platelets exit the spinning membrane separator 26 is equal to the difference between the flow rates of the PRP pump P4 and plasma pump P6. In an embodiment, the concentration of platelets in the platelet concentrate may be optically measured by sensor M4 (if present).

The valve V8 associated with valve station C8 is closed to prevent fluid flow through the line L158, thereby directing the flow of platelets along lines L161 and L162, through the valve station C7 associated with open valve V7, and into the platelet concentrate bag F6. The valve V8 associated with valve station C8 may be selectively opened to allow fluid flow through line L158 and to a junction, where it joins the plasma flowing through line L156 to the return bag F4, if necessary.

The draw phase may continue until the amount of blood drawn from the blood source reaches a target amount or the in-process bag F3 is filled to a particular level (as determined by a weight scale from which the in-process bag F3 is hung during the procedure) or until some other condition is satisfied.

During the draw phase, the controller 18 may continuously estimate the platelet concentration of a donor's circulating blood ($C_{donor}$) based on values obtained by the various sensors and scales and other inputs associated with the system and the equations described herein. By estimating the platelet concentration of a donor's circulating blood during a blood processing procedure, a platelet pre-count measurement is not required. Additionally, by continuously monitoring $C_{donor}$, controller 18 can be programmed to initiate an alarm or to alert a user when $C_{donor}$ drops below a pre-determined limit, for instance 100e3 platelets/pL. In an embodiment, if $C_{donor}$ drops below a pre-determined limit, controller 18 may alert the user and/or automatically end the procedure.

When the draw phase ends, the system transitions to one of two return phases, depending on whether only platelets are being collected or if both platelets and plasma are being collected. At least a portion of one of the separated blood components is conveyed to a recipient (which may be the blood source), while the collection of either platelets or both platelets and plasma proceeds.

During the return phase of a procedure in which only platelets are being collected, the anticoagulant pump P1 will stop drawing anticoagulant from the anticoagulant bag F1. The valve V10 associated with valve station C10 will close to prevent fluid flow through line L142 and the valve V11 associated with valve station C11 is opened to allow fluid flow through line L143. The valve V2 associated with valve station C2 also opens to allow flow through line L145, while the valve V1 associated with valve station C1 is closed to prevent flow through line L146.

With the valves so situated, the donor pump P2 will reverse direction to allow the contents of the return bag F4 to be conveyed to a recipient via the same needle used to draw blood into the fluid flow circuit 12. The return fluid (red blood cells and plasma) is pumped through lines L151 and L145, the valve station C2 associated with open valve V2, line L144, the sensor station S1 associated with pressure sensor A1, and into line L163. The return fluid travels along line L163, through a return line filter 58 and air detector M3 until it reaches a junction, which joins lines L163 and L164 (which leads to the saline bag F2). The valve V3 associated with valve station C3 is closed, thereby preventing fluid flow through line L164 and directing the return fluid further along line L163, through the valve station C11 associated with open valve V11, and along lines L143 and L140 to the recipient.

While fluid is being conveyed to the recipient, the blood in the in-process bag F3 acts as the blood supply for the centrifugal separator 16. When the system transitions to this return phase, the centrifuge pump P3 remains unchanged and separation continues in the same manner as described for the draw phase (i.e., with blood being separated in the centrifugal separation chamber 36 into red blood cells and platelet-rich plasma, the red blood cells flowing out of the centrifugal separation chamber 36 to the return bag F4, the platelet-rich plasma flowing to and being separated in the spinning membrane separator 26, and the separated platelets being collected while plasma is directed to the return bag F4) until the in-process bag F3 is emptied. Therefore, the system components downstream from the centrifuge pump P3 are "blinded" as to whether the system is in the draw phase or this return phase. It will be appreciated that a method as described herein is preferable to a batch process (by which blood is only separated during a draw phase and not during a return phase) because separation and collection may be continuous, thereby decreasing the time required to complete the procedure. Additionally, by continuously processing blood in the centrifugal separation chamber 36, the interface between the separated red blood cells and platelet-rich plasma is maintained, whereas the location of the interface is lost during the return phase of a batch procedure, which requires the interface to be reestablished during every draw/separation phase and further increases the duration of the procedure.

It will be seen that the separated plasma and red blood cells are conveyed to the return bag F4 at the same time that the contents of the return bag F4 are being conveyed to the recipient. The rate at which the donor pump P2 operates may be greater than the rate at which plasma and red blood cells are conveyed into the return bag F4 to allow the return bag F4 to empty during this return phase, even as separation continues. Once the in-process and/or return bag F3, F4 is empty, the system may transition back to the draw phase or to a final phase.

The return phase when collecting platelets and plasma is similar to the return phase when only platelets are being collected. The principal difference is that, when collecting platelets and plasma, the valve V5 associated with valve station C5 is open to allow flow through line L159, while the valve V4 associated with valve station C4 is closed to prevent flow through line L160, thereby directing the separated plasma exiting the spinning membrane separator 26 through the line L159 associated with open valve V5 to the plasma bag F9 instead of to the return bag F4. The valves may be so situated for the entire return phase or the conditions of valves V4 and V5 may be reversed for a portion of the phase (thereby directing separated plasma to the return bag F4 instead of the plasma bag F5), depending on the amount of plasma to be collected.

Once the in-process and/or return bag F3, F4 is empty, the system may transition back to the draw phase or to a final phase.

When the targeted amount of platelets (e.g., a single therapeutic dose of 3.3e11 platelets) or the targeted amounts of platelets and plasma have been collected, the blood source/recipient may be disconnected from the fluid flow circuit 12 and the system may transition to a final phase in which a platelet additive solution is added to the collected platelets. This may be carried out with the valves V9 and V7 associated with valve stations C9 and C7 (respectively) in an open condition to allow flow through lines L157 and L162, while the valves V8, V6, V5, and V4 associated with valve stations C8, C6, C5, and C4 (respectively) in a closed condition to prevent flow through lines L158, L155, L159, and L160. With the valves so situated, the plasma pump P6 may operate to draw the platelet additive solution from the PAS bag F7 via line L157, through the valve station C9 associated with open valve V9, line L156, spinner outlet sensor M2, the spinning membrane separator 26, lines L161 and L162, the valve station C7 associated with open valve V7, and into the platelet concentrate bag F6.

In an embodiment, the controller 18 may continuously estimate the platelet concentration of a donor's circulating blood ($C_{donor}$) throughout an entire blood processing procedure.

It shall be understood that the device, fluid circuit, and procedure described herein is exemplary and not meant to be limiting. The method of estimating the platelet concentration of a donor's circulating blood ($C_{donor}$) throughout a blood processing procedure may be used with a variety of blood processing devices and procedures without departing from the scope of the present disclosure. For example, the blood separation device 10 may be used with a fluid flow circuit having a pair of blood access devices (e.g., needles) for separating and collecting platelets or both platelets and plasma from blood. This allows for a single phase during which blood is simultaneous drawn from a blood source and processed, with a portion of at least one separated component being conveyed to a recipient. A more detailed disclosure of a double needle fluid flow circuit and procedure are disclosed in previously incorporated U.S. Patent No. 11,465,160. Accordingly, the controller 18 and method of estimating $C_{donor}$ described herein may be used with any known blood processing device and procedure without departing from the scope of the disclosure.

Other Aspects

There are additional aspects to the methods and systems described herein including, without limitation, the following aspects.

Aspect 1. A system for collecting platelets including: a reusable hardware unit, wherein the reusable hardware unit includes a separator; a disposable fluid circuit configured to be associated with the reusable hardware unit; and a controller configured to estimate a platelet concentration of a donor's circulating blood ($C_{donor}$).

Aspect 2. The system of Aspect 1, wherein $C_{donor}$ is based on a concentration of platelets in collected whole blood entering the separator ($C_{wbcent}$) and a percentage of collected whole blood in a separator inlet line (WB%).

Aspect 3. The system of Aspect 2, wherein $C_{donor}$ is obtained by determining a product of the concentration of platelets in collected whole blood entering the separator $C_{wbcent}$ and the percentage of collected whole blood in a separator inlet line (WB%), such that: $C_{donor} = C_{wbcent}*WB\%$.

Aspect 4. The system of Aspect 2, wherein the WB% is obtained by dividing an amount of collected whole blood in the separator inlet line with a total amount of collected whole blood and other fluid in the separator inlet line.

Aspect 5. The system of Aspect 3, wherein the other fluid is anticoagulant.

Aspect 6. The system of Aspect 4, wherein the WB% is obtained by dividing parts of collected whole blood with a sum of the parts of collected whole blood (Parts WB) and parts of anticoagulant, such that: WB% = Parts WB/(Parts WB + Parts Anticoagulant).

Aspect 7. The system of any one of Aspects 2 through 6, wherein $C_{wbcent}$ is based on a platelet concentration of separated platelet rich plasma ($C_{prp}$) and a hematocrit of collected whole blood entering the separator ($H_{wbcent}$).

Aspect 8. The system of Aspect 7, wherein $C_{prp}$ is measured with an optical sensor.

Aspect 9. The system of any one of Aspects 7 through 8, wherein $C_{wbcent}$ is determined by an equation: $C_{wbcent} = C_{prp}*(1 - H_{wbcent})$.

Aspect 10. The system of any one of Aspects 7 through 9, wherein $H_{wbcent}$ is based on a flow rate of separated packed red blood cells exiting the separator ($Q_{rbc}$), a flow rate of collected whole blood flowing into the separator ($Q_{wbcent}$), and a hematocrit of the separated packed red blood cells exiting the separator ($H_{rbc}$).

Aspect 11. The system of Aspect 10, wherein $H_{wbcent}$ is determined by dividing a product of Hrb, and Qrbc with Qwbcent, such that: $H_{wbcent} = (Hrbc*Qrbc)/Qwbcent$.

Aspect 12. The system of any one of Aspects 10 through 11, wherein $Q_{rbc}$ is based on the flow rate of collected whole blood flowing into the separator ($Q_{wbcent}$) and a flow rate of separated platelet rich plasma flowing out of the separator ($Q_{prp}$).

Aspect 13. The system of Aspect 12, wherein $Q_{wbcent}$ is based on a pump rate of a separator pump and $Q_{prp}$ is based on a pump rate of a platelet rich plasma pump.

Aspect 14. The system of Aspect 13, wherein $Q_{wbcent}$ and $Q_{prp}$ are predetermined values.

Aspect 15. The system of any one of Aspects 12 through 13, wherein $Q_{rbc}$ is determined by subtracting the flow rate of collected whole blood flowing into the separator ($Q_{wbcent}$) with the flow rate of separated platelet rich plasma flowing out of the separator ($Q_{prp}$), such that: $Q_{rbc} = Q_{wbcent} - Q_{prp}$.

Aspect 16. The system of any one of Aspects 10 through 15, wherein $H_{rbc}$ is a predetermined value.

Aspect 17. The system of any one of Aspects 10 through 15, wherein $H_{rbc}$ is determined by an optical sensor.

Aspect 18. The system of any one of Aspects 10 through 15, wherein $H_{rbc}$ is determined based on separator flow rates and separator g-force.

Aspect 19. The system of any one of Aspects 2 through 18, wherein the controller is configured to determine $C_{wbcent}$, WB%, $C_{prp}$, $H_{wbcent}$, $H_{rbc}$, and $Q_{rbc}$.

Aspect 20. The system of any one of Aspects 2 through 19, wherein the controller is configured to estimate the platelet concentration of a donor's circulating blood ($C_{donor}$) with an equation: $C_{donor} = (C_{prp}*WB\%)*(1 - (H_{rbc}*Q_{rbc})/Q_{wbcent}))$.

Aspect 21. The system of any one of Aspects 2 through 20, wherein the separator is a centrifuge.

Aspect 22. A method for processing whole blood from a donor including: collecting whole blood from a donor; separating the collected whole blood into its constituent blood components including separated platelets; collecting the separated platelets; and estimating a concentration of platelets of a donor's blood ($C_{donor}$) during processing.

Aspect 23. The method of Aspect 22, further including connecting a donor to a blood processing system, wherein the processing system includes: a reusable hardware unit, wherein the reusable hardware unit includes a separator; a disposable fluid circuit configured to be associated with the reusable hardware unit; and a controller configured to estimate the platelet concentration of a donor's circulating blood ($C_{donor}$).

Aspect 24. The method of any one of Aspects 22 through 23, wherein $C_{donor}$ is based on a concentration of platelets in collected whole blood entering the separator ($C_{wbcent}$) and a percentage of collected whole blood in a separator inlet line (WB%).

Aspect 25. The method of Aspect 24, wherein $C_{donor}$ is obtained by determining a product of the concentration of platelets in collected whole blood entering the separator $C_{wbcent}$ and the percentage of collected whole blood in a separator inlet line (WB%), such that: $C_{donor} = C_{wbcent}*WB\%$.

Aspect 26. The method of Aspect 25, wherein the WB% is obtained by dividing an amount of collected whole blood in the separator inlet line with a total amount of collected whole blood and other fluid in the separator inlet line.

Aspect 27. The method of Aspect 26, wherein the other fluid is anticoagulant.

Aspect 28. The method of Aspect 27, wherein the WB% is obtained by dividing parts of collected whole blood (Parts WB) with a sum of the parts of collected whole blood and parts of anticoagulant, such that: WB% = Parts WB/(Parts WB + Parts Anticoagulant).

Aspect 29. The method of any one of Aspects 22 through 28, wherein $C_{wbcent}$ is based on a platelet concentration of separated platelet rich plasma ($C_{prp}$) and a hematocrit of collected whole blood entering the separator ($H_{wbcent}$).

Aspect 30. The method of Aspect 29, wherein $C_{prp}$ is measured with an optical sensor.

Aspect 31. The method of any one of Aspects 29 through 30, wherein $C_{wbcent}$ is determined by an equation: $C_{wbcent} = C_{prp}*(1 - H_{wbcent})$.

Aspect 32. The method of any one of Aspects 29 through 31, wherein $H_{wbcent}$ is based on a flow rate of separated packed red blood cells exiting the separator ($Q_{rbc}$), a flow rate of collected whole blood flowing into the separator ($Q_{wbcent}$), and a hematocrit of the separated packed red blood cells exiting the separator ($H_{rbc}$).

Aspect 33. The method of Aspect 32, wherein $H_{wbcent}$ is determined by dividing a product of Hrb, and $Q_{rbc}$ with $Q_{wbcent}$, such that: $H_{wbcent} = (H_{rbc}*Q_{rbc})/Q_{wbcent}$.

Aspect 34. The method of any one of Aspects 32 through 33, wherein $Q_{rbc}$ is based on the flow rate of collected whole blood flowing into the separator ($Q_{wbcent}$) and a flow rate of separated platelet rich plasma flowing out of the separator ($Q_{prp}$).

Aspect 35. The method of Aspect 34, wherein $Q_{wbcent}$ is based on a pump rate of a separator pump and $Q_{prp}$ is based on a pump rate of a platelet rich plasma pump.

Aspect 36. The method of Aspect 35, wherein $Q_{wbcent}$ and $Q_{prp}$ are predetermined values.

Aspect 37. The method of any one of Aspects 34 through 35, wherein $Q_{rbc}$ is determined by subtracting the flow rate of collected whole blood flowing into the separator ($Q_{wbcent}$) with the flow rate of separated platelet rich plasma flowing out of the separator ($Q_{prp}$), such that: $Q_{rbc} = Q_{wbcent} - Q_{prp}$.

Aspect 38. The method of any one of Aspects 32 through 37, wherein $H_{rbc}$ is a predetermined value.

Aspect 39. The method of any one of Aspects 32 through 37, wherein $H_{rbc}$ is determined by an optical sensor.

Aspect 40. The method of any one of Aspects 32 through 37, wherein $H_{rbc}$ is determined based on separator flow rates and separator g-force.

Aspect 41. The method of any one of Aspects 23 through 40, wherein the controller is configured to determine $C_{wbcent}$, WB%, $C_{prp}$, $H_{wbcent}$, $H_{rbc}$, and $Q_{rbc}$.

Aspect 42. The method of any one of Aspects 23 through 41, wherein the controller is configured to estimate the platelet concentration of a donor's circulating blood ($C_{donor}$) with an equation: $C_{donor} = (C_{prp}*WB\%)*(1 - (H_{rbc}*Q_{rbc})/Q_{wbcent}))$.

Aspect 43. The method of any one of Aspects 23 through 42, wherein the separator is a centrifuge.

It will be understood that the embodiments and examples described above are illustrative of some of the applications or principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that the claims may be directed to the features thereof, including as combinations of features that are individually disclosed or claimed herein.

**Claims**

1. A system for collecting platelets comprising:

   a reusable hardware unit, wherein the reusable hardware unit comprises a separator;
   a disposable fluid circuit configured to be associated with the reusable hardware unit; and
   a controller configured to estimate a platelet concentration of a donor's circulating blood ($C_{donor}$).

2. The system of claim 1, wherein $C_{donor}$ is based on a concentration of platelets in collected whole blood entering the separator ($C_{wbcent}$) and a percentage of collected whole blood in a separator inlet line (WB%).

3. The system of claim 2, wherein $C_{donor}$ is obtained by determining a product of the concentration of platelets in collected whole blood entering the separator $C_{wbcent}$ and the percentage of collected whole blood in a separator inlet line (WB%), such that:

$$C_{donor} = C_{wbcent} * WB\%.$$

4. The system of claim 2, wherein the WB% is obtained by dividing an amount of collected whole blood in the separator inlet line with a total amount of collected whole blood and other fluid, in particular anticoagulant, in the separator inlet line.

5. The system of claim 3, wherein the WB% is obtained by dividing parts of collected whole blood with a sum of the parts of collected whole blood (Parts WB) and parts of anticoagulant, such that:

$$WB\% = Parts\ WB/(Parts\ WB + Parts\ Anticoagulant).$$

6. The system of any one of claims 2-5, wherein $C_{wbcent}$ is based on a platelet concentration of separated platelet rich plasma ($C_{prp}$) and a hematocrit of collected whole blood entering the separator ($H_{wbcent}$).

7. The system of claim 6, wherein $C_{wbcent}$ is determined by an equation:

$$C_{wbcent} = C_{prp} * (1 - H_{wbcent}).$$

8. The system of any one of claims 6-7, wherein $H_{wbcent}$ is based on a flow rate of separated packed red blood cells exiting the separator ($Q_{rbc}$), a flow rate of collected whole blood flowing into the separator ($Q_{wbcent}$), and a hematocrit of the separated packed red blood cells exiting the separator ($H_{rbc}$).

9. The system of claim 8, wherein $H_{wbcent}$ is determined by dividing a product of $H_{rbc}$ and $Q_{rbc}$ with $Q_{wbcent}$, such that:

$$H_{wbcent} = (H_{rbc} * Q_{rbc})/Q_{wbcent}.$$

10. The system of any one of claims 8-9, wherein $Q_{rbc}$ is based on the flow rate of collected whole blood flowing into the separator ($Q_{wbcent}$) and a flow rate of separated platelet rich plasma flowing out of the separator ($Q_{prp}$), wherein $Q_{rbc}$ is determined by subtracting $Q_{prp}$ from $Q_{wbcent}$ such that: $Q_{rbc} = Q_{wbcent} - Q_{prp}$.

11. The system of claim 10, wherein $Q_{wbcent}$ is based on a pump rate of a separator pump and $Q_{prp}$ is based on a pump rate of a platelet rich plasma pump.

**12.** The system of any one of claims 8-11, wherein $H_{rbc}$ is a predetermined value.

**13.** The system of any one of claims 8-11, wherein $H_{rbc}$ is determined by an optical sensor, based on separator flow rates and separator g-force..

**14.** The system of any one of claims 2-13, wherein the controller is configured to determine $C_{wbcent}$, WB%, $C_{prp}$, $H_{wbcent}$, $H_{rbc}$, and $Q_{rbc}$.

**15.** The system of any one of claims 2-14, wherein the controller is configured to estimate the platelet concentration of a donor's circulating blood ($C_{donor}$) with an equation:

$$C_{donor} = (C_{prp}*WB\%)*(1 - (H_{rbc}*Q_{rbc})/Q_{wbcent})).$$

**FIG. 1**

**FIG.2**

FIG. 3

70

72

Measure PRP or PC concentration
$(C_{prp}$ or $C_{pc})$

74

Determine flow rates
$Q_{WBcent}, Q_{RBC}, Q_{PRP}, , Q_{PC}$

76

Determine packed red blood cell
hematocrit
$(H_{RBC})$

78

Calculate the donor PLT
concentration
$C_{donor}$

FIG. 4

Europäisches
Patentamt

European
Patent Office

Office européen
des brevets

## EUROPEAN SEARCH REPORT

**Application Number**

EP 24 21 4463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 065 495 A2 (TERUMO CORP [JP]) 3 January 2001 (2001-01-03) * figures 1-7 * * paragraph [0001] - paragraph [0002] * * paragraph [0010] * * paragraph [0013] - paragraph [0015] * * paragraph [0018] - paragraph [0019] * * paragraph [0059] * * paragraph [0119] - paragraph [0121] * * the whole document *<br><br>- - - - - | 1-15 | INV. A61M1/36 A61M1/38 |
| X | US 2003/066807 A1 (SUZUKI ATSUSHI [JP]) 10 April 2003 (2003-04-10) * figures 1-3 * * figures 5-16 * * paragraph [0002] - paragraph [0003] * * paragraph [0008] * * paragraph [0090] * * paragraph [0117] * * the whole document *<br><br>- - - - - | 1-15 | |

|  |  |
|---|---|
|  | **TECHNICAL FIELDS SEARCHED (IPC)**<br><br>A61M |

The present search report has been drawn up for all claims

| Place of search | Date of completion of the search | Examiner |
|---|---|---|
| The Hague | 13 March 2025 | Ugarte, Eva |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

# EP 4 559 492 A1

## ANNEX TO THE EUROPEAN SEARCH REPORT
## ON EUROPEAN PATENT APPLICATION NO.

EP 24 21 4463

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2025

| Patent document cited in search report | | Publication date | Patent family member(s) | | Publication date |
|---|---|---|---|---|---|
| EP 1065495 | A2 | 03-01-2001 | AT | E317113 T1 | 15-02-2006 |
| | | | DE | 60025774 T2 | 12-10-2006 |
| | | | EP | 1065495 A2 | 03-01-2001 |
| | | | JP | 3817091 B2 | 30-08-2006 |
| | | | JP | 2001021479 A | 26-01-2001 |
| | | | US | 6678040 B1 | 13-01-2004 |
| US 2003066807 | A1 | 10-04-2003 | AT | E532543 T1 | 15-11-2011 |
| | | | EP | 1295619 A2 | 26-03-2003 |
| | | | JP | 4832683 B2 | 07-12-2011 |
| | | | JP | 2003088581 A | 25-03-2003 |
| | | | US | 2003066807 A1 | 10-04-2003 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 11465160 B
- US 5194145 A
- US 5868696 A
- US 6419822 B
- US 8556793 B
- US 10127523